# EUROPEAN PATENT SPECIFICATION

(11) **EP 1 276 865 B1**
(45) Date of publication and mention of the grant of the patent: **21.05.2014**
(21) Application number: 01928946.1
(22) Date of filing: 27.04.2001
(51) Int. Cl.: C12N 15/12, C12N 15/11, C12N 15/62, C07K 14/47, C07K 14/71, C07K 14/72, C12Q 1/68, G01N 33/53, G01N 33/68, C12N 15/10

(54) **METHODS FOR BINDING AN EXOGENOUS MOLECULE TO CELLULAR CHROMATIN**
VERFAHREN ZUR BINDUNG EINES EXOGENEN MOLEKÜLS AN ZELLULÄRES CHROMATIN
METHODES DE FIXATION D'UNE MOLECULE EXOGENE A LA CHROMATINE CELLULAIRE

(30) Priority: 28.04.2000 US 200590 P
(43) Date of publication of application: 22.01.2003
(73) Proprietor: Sangamo BioSciences, Inc., Richmond, CA 94804 (US)
(72) Inventor: RASCHKE, Eva, Berkeley, CA 94703 (US); WOLFFE, Alan, P., Orinda, CA 94563 (US); CASE, Casey, C., San Mateo, CA 94022 (US)
(74) Representative: Weiss, Wolfgang
(86) International application number: PCT/US2001/013631
(87) International publication number: WO 2001/083751

(56) References cited:
- WO-A-98/53057
- WO-A-99/48909
- US-A- 5 306 619
- ZHANG LEI ET AL: "Synthetic zinc finger transcription factor action at an endogenous chromosomal site. Activation of the human erythropoietin gene" JOURNAL OF BIOLOGICAL CHEMISTRY, AMERICAN SOCIETY OF BIOLOGICAL CHEMISTS, BALTIMORE, MD, US, vol. 275, no. 43, 27 October 2000 (2000-10-27), pages 33850-33860, XP002170053 ISSN: 0021-9258
- LIU P-Q ET AL: "REGULATION OF AN ENDOGENOUS LOCUS USING A PANEL OF DESIGNED ZINC FINGER PRTEINS TARGETED TO ACESSIBLE CHROMATIN REGIONS ACTIVATION OF VASCULAR ENDOTHELIAL GROWTH FACTOR A" JOURNAL OF BIOLOGICAL CHEMISTRY, AMERICAN SOCIETY OF BIOLOGICAL CHEMISTS, BALTIMORE, MD, US, vol. 276, no. 14, 6 April 2001 (2001-04-06), pages 11323-11334, XP000999288 ISSN: 0021-9258
- TAYLOR A. ET AL: "The anti-cancer agent distamycin A displaces essential transcription factors and selectively inhibits myogenic differentiation", MOLECULAR AND CELLULAR BIOCHEMISTRY, vol. 169, 1997, pages 61-72,
- RICCIO ET AL: "Mediation by a CREB family transcription factor of NGF-dependent survival of sympathetic Neurons", SCIENCE, vol. 286, 17 December 1999 (1999-12-17), pages 2358-2361,

## Description

### TECHNICAL FIELD

The present disclosure is in the field of gene regulation, specifically, regulation of an endogenous gene in a cell and methods of regulating an endogenous gene through binding of an exogenous molecule.

### BACKGROUND

Regulation of gene expression in a cell is often mediated by sequence-specific binding of gene regulatory proteins. These regulatory proteins can effect either positive or negative regulation of gene expression. Generally, a regulatory protein will exhibit preference for binding to a particular binding sequence, or target site. Target sites for many regulatory proteins (and other molecules) are known or can be determined by one of skill in the art.

Recently, it has become possible to obtain regulatory proteins which bind to predetermined DNA target sites. Such proteins can be obtained, for example, by using a specific DNA sequence for selection of a binding protein from a pool of proteins having fully or partially randomized sequence at certain amino acid residues; or through design of a protein having an amino acid sequence known to bind a particular target site, using design concepts that relate the amino acid sequence of the protein to the DNA sequence of the target site. This technology is most highly developed for the class of DNA-binding proteins known as zinc finger proteins (ZFPs). *See,* for example, U.S. Patents 5,789,538; 6,007,988; 6,013,453; WO 95/19431; WO 98/54311; WO 00/42219; Rebar et al. (1994) Science 263:671-673; Jamieson et al.(1994) Biochemistry 33:5689-5695; Choo et al. (1994) Proc. Natl. Acad. Sci USA 91:11163-11167; and Greisman et al. (1997) Science 275:657-661.

Recombinant ZFPs, selected or designed by the methods described above, are reported to have the ability to regulate expression of transiently expressed reporter genes and randomly integrated exogenous target genes in cultured cells. For example, a ZFP DNA-binding domain can be fused to a transcriptional activation domain (such as, for example, VP16 or VP64) or a transcriptional repression domain (such as, for example, KRAB, ERD, or SID) to obtain activation or repression, respectively, of a gene adjacent to a target sequence for the ZFP DNA-binding domain. *See,* for example, Choo et al. (1994) Nature 372:642-645; Pomerantz et al. (1995) Science 267:93-96; Liu et al. (1997) Proc. Natl. Acad. Sci. USA 94:5525-5530; and Beerli et al. (1998) Proc. Natl. Acad. Sci. USA 95:14628-14633.

Kang et al. (2000) J. Biol. Chem. 275:8742-8748 report the effects of cellular expression of engineered ZFPs on the transcription of extrachromosomal and integrated reporter genes. They reported that an engineered ZFP was able to override transcriptional activation of a reporter gene by a GAL4-VP16 fusion protein. These authors did not disclose a method for selecting a binding site for an exogenous molecule in cellular chromatin.

Beerli et al. (2000) Proc. Natl. Acad. Sci. USA 97:1495-1500 report regulation of endogenous *erbB2* and *erbB3* genes with designed ZFPs. However, they do not disclose methods for selecting a binding site for an exogenous molecule in cellular chromatin.

Zhang et al. (2000) J. Biol. Chem. 43:33850-33860 disclose targeting the activation of an endogenous chromosomal locus including the human erythropoietin gene using synthetic transcription factors, wherein the transcription factors are targeted to particular DNA sequences through engineering of a zinc finger binding domain.

Despite the advances in the selection and design of sequence-specific DNA binding gene regulatory proteins, their application to the regulation of an endogenous cellular gene can, in some cases, be limited if their access to the target site is restricted in the cell. Possible sources of restricted access could be related to one or more aspects of the chromatin structure of the gene.

Cellular DNA, including the cellular genome, generally exists in the form of chromatin, a complex comprising nucleic acid and protein. Indeed, most cellular RNAs also exist in the form of nucleoprotein complexes. The nucleoprotein structure of chromatin has been the subject of extensive research, as is known to those of skill in the art. In general, chromosomal DNA is packaged into nucleosomes. A nucleosome comprises a core and a linker. The nucleosome core comprises an octamer of core histones (two each of H2A, H2B, H3 and H4) around which is wrapped approximately 150 base pairs of chromosomal DNA. In addition, a linker DNA segment of approximately 50 base pairs is associated with linker histone H1. Nucleosomes are organized into a higher-order chromatin fiber and chromatin fibers are organized into chromosome. *See*, for example, Wolffe "Chromatin: Structure and Function" 3rd Ed., Academic Press, San Diego, 1998.

Due to the fact that cellular DNAs (and, hence, cellular genes) are packaged in chromatin, the presence of a target site in a cellular nucleic acid does not necessarily guarantee that binding will occur, in a cell, between the sequence of the target site and a molecule capable of binding to it. For example, the structure of the cellular chromatin in which the target site is packaged may serve to occlude or otherwise block the target site, limiting the accessibility of binding molecules, such as transcription factors, to the target site.

Accordingly, it would be useful to have additional methods of identifying accessible target sites (*i.e*., binding sites) for exogenous molecules in cellular chromatin and additional methods for binding an exogenous molecule to a binding site within a region of interest in cellular chromatin.

### SUMMARY

Subject of the present invention is a cell comprising a complex between an exogenous zinc finger DNA binding protein and chromosomal cellular chromatin;
wherein the exogenous zinc finger DNA binding protein is designed and/or selected to specifically bind to a target site in a region of the cellular chromatin that is sensitive to digestion with DNAse I; and
wherein the exogenous zinc finger DNA binding protein is bound to the target site.

Methods for binding an exogenous molecule to a binding site in cellular chromatin are provided. The binding site can be in any region of interest in the cellular chromatin, including transcribed, non-transcribed, coding and/or non-coding regions. Cellular chromatin can comprise, for example, a chromosome, episome, or any other cellular nucleic acid. The methods comprise identification, within the region of interest, of an accessible region in the cellular chromatin, identification of a target site for the exogenous molecule within the accessible region, and introduction of the exogenous molecule into the cell, whereby it binds to the binding site.

In one embodiment, the method also comprises testing for the binding of the exogenous molecule to the binding site, using methods such as, for example, chromatin immunoprecipitation and/or in vivo footprinting.

Also disclosed herein are methods for identifying a binding site for an exogenous molecule within a region of interest in cellular chromatin, wherein the methods comprise identification of an accessible region in the cellular chromatin and identification of a target site for the exogenous molecule within the accessible region. In additional embodiments, the methods can further comprise introducing the exogenous molecule into the cell and testing for the binding of the exogenous molecule to the binding site. Testing for binding can be conducted using methods such as, for example, chromatin immunoprecipitation and/or in vivo footprinting.

Accessible regions are determined, for example, by identifying regions in cellular chromatin that are hypersensitive to the action of various structural probes, either chemical or enzymatic. In a preferred embodiment, an enzymatic probe is used. In a more preferred embodiment the enzymatic probe is deoxyribonuclease I (DNase I).

A number of different types of exogenous molecules can be bound to a binding site in cellular chromatin using the methods disclosed herein. These include, but are not limited to, macromolecules (*e.g*., proteins, nucleic acids), small molecules, nucleic acid analogues such as peptide nucleic acids, (PNAs), DNA-RNA hybrids, DNA-RNA chimeras, PNA-DNA chimeras, PNA-RNA chimeras, PNA-DNA-RNA-chimeras, and protein analogues such as, for example, polyamides and peptide analogues which bind in the major and/or minor groove of double-stranded DNA such as, for example, distamycin and bleomycin.

In certain embodiments, when the exogenous molecule is a protein, the protein can be one that participates in one or more of the following processes: replication, recombination, integration, DNA repair, transcriptional regulation or chromatin remodeling. Transcriptional regulation can include processes such as gene activation and gene repression. Gene activation can include increases in transcription above a basal level, or relief of the total transcriptional repression of a gene. Similarly, transcriptional repression can include decreases in transcription of an activated gene to a low but detectable level, or complete silencing of transcription. Chromatin remodeling includes processes such as those which effect changes in the acetylation, phosphorylation, methylation, ubiquitination and/or ADP-ribosylation state of histones, and/ or proteolysis of histones. Chromatin remodeling can also result from the action of enzymes or enzyme complexes such as DNA and RNA polymerases, topoisomerases, and complexes such as the SWI/SNF complex. Any change in the activity of a gene, regardless of the cause of the change, can be described as a modulation of gene expression.

In a further embodiment, an exogenous molecule is a protein and the protein is a transcription factor. In a preferred embodiment, the transcription factor is a zinc finger protein (ZFP). ZFP transcription factors and their target sites are described, for example, in U.S. Patent No. 5,789,538; U.S. Patent No. 6007,408; U.S. Patent No. 6,013,453; PCT WO 95/19431; PCT WO 98/54311 co-owned WO 00/42219 and references cited therein. In one embodiment, the binding site for a ZFP comprises the sequence 5'-NNx aNy bNz c-3', wherein each of (x,a), (y,b) and (z,c) is (N,N) or (G,K) and at least one of (x,a), (y,b) and (z,c) is (G,K); wherein N is any nucleotide and K is either G or T.

In another embodiment, an accessible region is identified within a region of interest and a ZFP target site is located within the accessible region. A ZFP that binds to the target site is designed. The designed ZFP can be introduced into the cell, or a nucleic acid encoding the designed ZFP can be designed and the designed nucleic acid can be introduced into the cell, where it will express the designed ZFP. Methods for the design and/or selection of ZFPs that bind specific sequences are disclosed in U.S. Patent No. 5,789,538; U.S. Patent No. 6007,408; U.S. Patent No 6,013 453; PCT WO 95/19431; PCT WO 98/54311 co-owned WO 00/42219 and references cited therein. Methods for selection include, but are not limited to, phage display and *in vivo* selection.

In another embodiment, when the exogenous molecule is a protein, the protein is used for detection of one or more target sequences.

An exogenous molecule can be introduced into a cell by any method that is known to one of skill in the art including, but not limited to, lipid-mediated gene transfer (*e.g*., liposomes), electroporation, direct injection, particle bombardment, calcium phosphate co-precipitation, DEAE-dextran mediated transfer and viral vector-mediated gene transfer. *See* also Ausubel et al., Current Protocols in Molecular Biology, John Wiley & Sons, Inc., 1987 and periodic supplements (especially Chapter 9); Sambrook et al., Molecular Cloning: A Laboratory Manual, 2nd edition, Cold Spring Harbor Laboratory Press, Cold Spring Harbor, New York, 1989 (especially Chapter 16); and related references.

In additional embodiments, when the exogenous molecule is a protein, the protein is encoded by an exogenous nucleic acid. In these embodiments the exogenous nucleic acid is introduced into the cell, wherein it encodes an exogenous protein.

The methods disclosed herein are applicable to any cell type including, but not limited to, prokaryotic cells, eukaryotic cells, Archaea and Mycoplasma. Eucaryotic cells include, but are not limited to, fungal cells, plant cells and animal cells, including mammalian cells and, in particular, human cells.

Binding sites for a number of different types of exogenous molecules can be identified using the methods disclosed herein. These include, but are not limited to, macromolecules (*e.g*., proteins, nucleic acids), small molecules, nucleic acid analogues such as peptide nucleic acids, (PNAs), DNA-RNA hybrids, DNA-RNA chimeras, PNA-DNA chimeras PNA-RNA chimeras, PNA-DNA-RNA chimeras, protein analogues such as, for example, polyamides and peptide analogues which bind in the major and/or minor groove of double-stranded DNA such as, for example, distamycin and bleomycin.

In methods comprising introduction of an exogenous molecule into a cell and testing for binding of the exogenous molecule to a binding site, a ZFP that binds to a target site, located within an accessible region, is designed. The designed ZFP can be introduced into the cell, or a nucleic acid encoding the designed ZFP can be designed and the designed nucleic acid can be introduced into the cell, where it will express the designed ZFP. Methods for the design and/or selection of ZFPs that bind specific sequences are disclosed in U.S. Patent No. 5,789,538; U.S. Patent No. 6007,408; U.S. Patent No. 6,013,453; PCT WO 95/19431; PCT WO 98/54311 co-owned WO 00/42219 and references cited therein. Methods for selection include, but are not limited to, phage display and in vivo selection.

In another embodiment, when the exogenous molecule is a protein, the protein is used for detection of a target sequence.

In additional embodiments, when the exogenous molecule is a protein, the protein is encoded by an exogenous nucleic acid. In these embodiments the exogenous nucleic acid is introduced into the cell, wherein it encodes an exogenous protein.

Methods disclosed herein for identifying a binding site are applicable to binding sites in any cell type including, but not limited to, prokaryotic cells, eukaryotic cells, Archaea and Mycoplasma. Eucaryotic cells include, but are not limited to, fungal cells, plant cells and animal cells, including mammalian cells and, in particular, human cells.

Also disclosed herein are complexes between an exogenous molecule and a binding site, as well as cells comprising a complex between an exogenous molecule and a binding site, wherein the binding site is located within a region of interest in cellular chromatin and wherein the binding site is determined according to the methods disclosed herein.

### BRIEF DESCRIPTION OF THE DRAWINGS

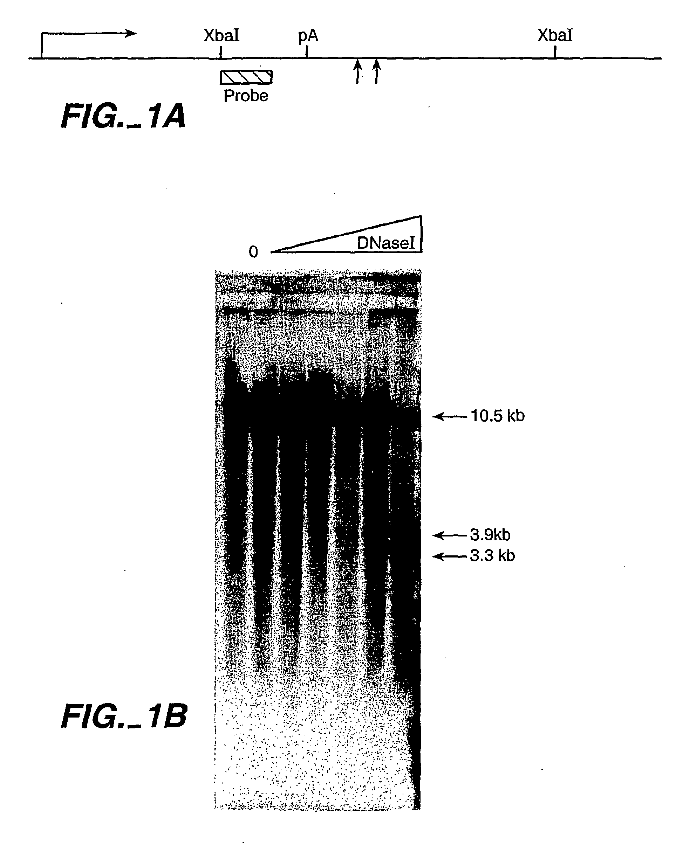

**Figure 1** shows an analysis of DNase hypersensitive sites in the human erythropoietin gene in 293 cells. Figure 1A shows a schematic diagram of the structure of the gene, indicating the transcriptional start site (rightward-pointing arrow), the transcription termination site (pA), and the locations of Xba I sites which define the DNA fragment used for mapping. Shown below the line are the location of the probe (a ³²P-labeled Xba I-Kpn I fragment, hatched box) and the locations of two DNase hypersensitive sites (upward-pointing arrows). Figure 1B shows a phosphorimager image of a 1% agarose gel. Locations of the positions of migration of the XbaI fragment (10.5 kb) and the two fragments defined by the DNase hypersensitive sites (3.9 kb and 3.3 kb) are shown to the right of the gel image.

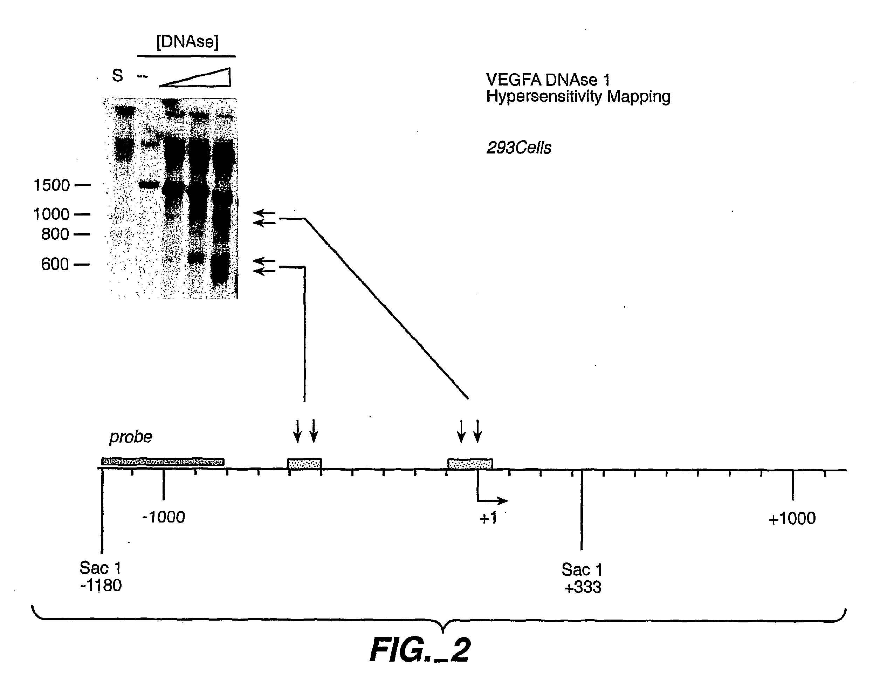

**Figure 2** shows an analysis of DNase hypersensitive sites in the human VEGF-A gene in 293 cells.

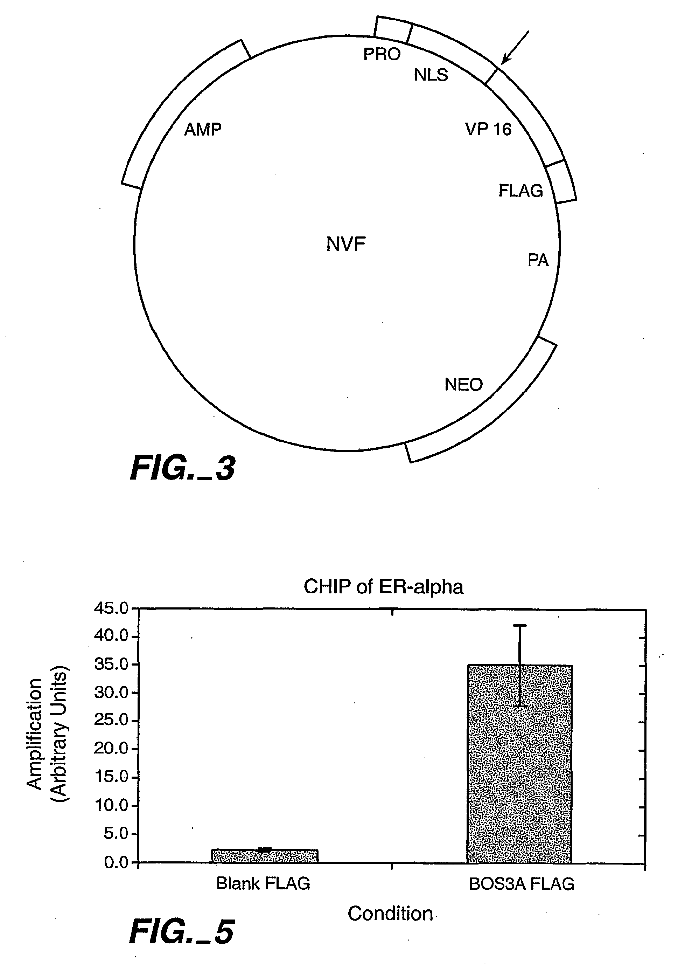

**Figure 3** shows a schematic diagram of the NVF plasmid. Regions of plasmid sequence encoding a CMV promoter (PRO), a nuclear localization signal (NLS), a transcriptional activation domain (VP16), a FLAG epitope (FLAG), a bovine growth hormone polyadenylation signal (pA), and resistance to neomycin (NEO) and ampicillin (AMP) are indicated. The arrow indicates the region at which ZFP-encoding sequences are inserted to generate the VEGF 1 and VEGF 3a/1 plasmids. The drawing is not to scale.

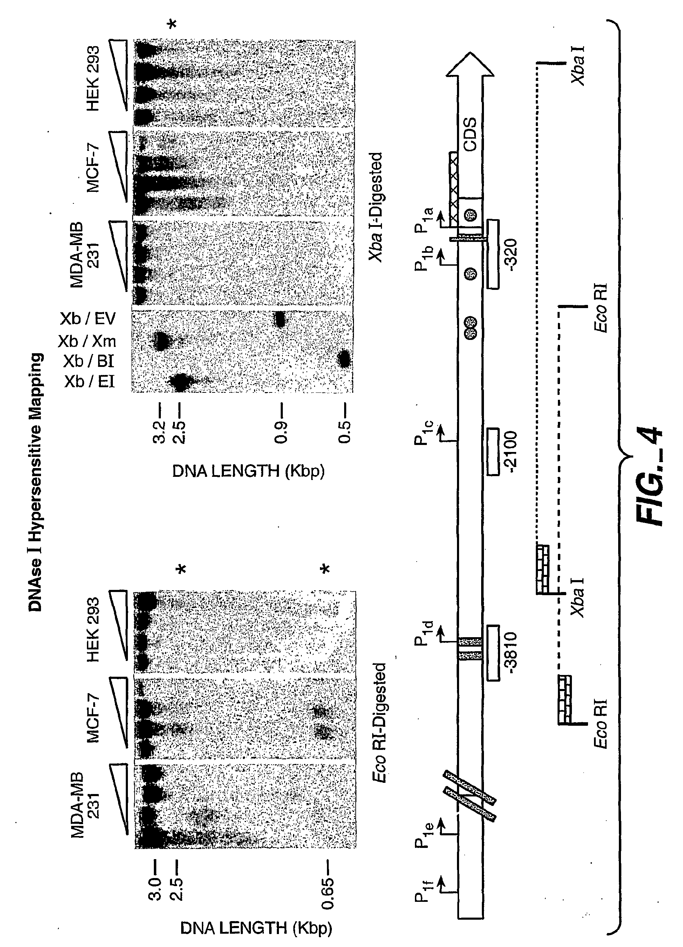

**Figure 4** shows ER-alpha hypersensitive site mapping. The gels at the top of the figure show digestion of chromatin from different cell lines (as indicated above gel) with increasing concentrations of DNase I (indicated by triangles). Molecular weight markers are also shown. At the bottom of the figure, a schematic diagram of the upstream region of the ER-alpha gene shows locations of promoters (indicated by P), DNase-hypersensitive regions (-3810, -2100 and -320), and the Eco RI and Xba I fragments used as probes for DNase-hypersensitive region analysis.
**Figure 5** shows analysis, by chromatin immunoprecipitation, of binding of an exogenous molecule to the ER-alpha gene. See Example 15.

### DETAILED DESCRIPTION

In many instances in the areas of, for example, therapeutics, diagnostics, target validation and research, the ability to regulate an endogenous gene using an exogenous molecule would be desirable. For example, many pathophysiological processes are the result of aberrant gene expression. Examples include the inappropriate activation of proinflammatory cytokines in rheumatoid arthritis, under-expression of the hepatic LDL receptor in hypercholesteremia, over-expression of proangiogenic factors, and under-expression of antiangiogenic factors in solid tumor growth. If therapeutic methods for control of gene expression existed, many of these pathologies could be more optimally treated.

In another example of the therapeutic utility of being able to regulate cellular gene expression, developmentally silent or otherwise inactive genes are activated in order to treat a particular disease state. Examples of possible therapeutic applications of gene reactivation include activation of developmentally silent fetal globin genes to treat sickle cell disease and the activation of the dystrophin and/or eutrophin genes to treat muscular dystrophy. In addition, pathogenic organisms such as viruses, bacteria, fungi, and protozoa could be controlled by altering gene expression. Accordingly, there is a need for improved therapeutic approaches that act through sequence-specific regulation of disease-related genes.

One way in which regulation of an endogenous gene can be accomplished is through the use of a transcriptional regulatory protein which binds to DNA. For example, one can search a nucleotide sequence comprising the gene of interest for the presence of a binding sequence for a transcriptional regulatory protein (i.e., a target site) and, if such a sequence is found, introduce the transcriptional regulatory protein into the cell. However, the presence of a target site within or adjacent to the sequence of a gene does not always imply that a protein which recognizes that sequence will bind to the sequence as present in cellular chromatin. There are several reasons why this might be the case. First, the target site may be blocked by histones or other chromosomal proteins. Second, the DNA sequence of the target site may have a secondary or tertiary structure that is incompatible with binding. For example, the wrapping of DNA around a nucleosome can affect the secondary and tertiary structure of DNA. In addition, certain DNA-binding proteins are know to bend or kink DNA; such bending or kinking may be required for regulatory functions of DNA to be manifested. Third, the binding site for a regulatory protein may be defined by both nucleic acid and protein surfaces.

Thus, although in certain circumstances it may be possible for a binding molecule to interact with its target site in cellular chromatin; in other situations, binding of a molecule to its target site, as present in cellular chromatin, may not occur due to one or more aspects of chromatin structure. Accordingly, methods for determining whether a target site for a binding molecule is also a binding site in cellular chromatin are disclosed herein.

### General

The practice of the methods described herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. *See*, for example, Sambrook et al. MOLECULAR CLONING: A LABORATORY MANUAL, Second edition, Cold Spring Harbor Laboratory Press, 1989; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; and Wolffe, CHROMATIN STRUCTURE AND FUNCTION, Third edition, Academic Press, San Diego, 1998.

### Definitions

Chromatin is the nucleoprotein structure comprising the cellular genome. Cellular chromatin comprises nucleic acid, primarily DNA, and protein, including histones and non-histone chromosomal proteins.

A chromosome, as is known to one of skill in the art, is a chromatin complex comprising all or a portion of the genome of a cell. The genome of a cell is often characterized by its karyotype, which is the collection of all the chromosomes that comprise the genome of the cell. The genome of a cell can comprise one or more chromosomes.

An episome is a replicating nucleic acid, nucleoprotein complex or other structure comprising a nucleic acid that is not part of the chromosomal karyotype of a cell. Examples of episomes include plasmids and certain viral genomes.

A target site is a nucleic acid sequence that defmes a portion of a nucleic acid to which a binding molecule will bind, provided sufficient conditions for binding exist. For example, the sequence 5'-GAATTC-3' is a target site for the Eco RI restriction endonuclease. Binding of a molecule to its target site will generally occur in a naked nucleic acid molecule, for example, EcoRI binds to (and cleaves at) its target site in naked DNA. However, a target site present in cellular chromatin might be blocked as a result of some aspect of chromatin structure and thus inaccessible to its binding molecule. In other cases, factors in addition to a target site may be required for binding of a molecule to a nucleic acid at the target site. For instance, binding of a molecule to a polynucleotide comprising a target site may require both a particular nucleotide sequence and a particular protein composition adjacent to, or in the vicinity of, the target site. Conditions such as, for example, temperature, pH, and ionic strength can also affect binding of a molecule to its target site.

A binding site in cellular chromatin is a region at which a particular molecule, for example a protein, will bind to a target site in the chromatin. A binding site will generally comprise a target site, but not every target site will constitute a binding site in cellular chromatin. For example, a target site may be occluded by one or more chromosomal components, such as histones or nonhistone proteins, or might be rendered inaccessible to its binding molecule because of nucleosomal or higher-order chromatin structure. On the other hand, the presence of one or more chromosomal proteins may be required, in addition to a target site, to define a binding site.

An accessible region is a site in a chromosome, episome or other cellular structure comprising a nucleic acid, in which a target site present in the nucleic acid can be bound by an exogenous molecule which recognizes the target site. Without wishing to be bound by any particular theory, it is believed that an accessible region is one that is not packaged into a nucleosomal structure. The distinct structure of an accessible region can often be detected by its sensitivity to chemical and enzymatic probes, for example, nucleases.

An endogenous molecule is one that is normally present in a cell. For example, an endogenous nucleic acid can comprise a chromosome, the genome of a mitochondrion, chloroplast or other organelle, or a naturally-occurring episomal nucleic acid.

An exogenous molecule is a molecule that is not normally present in a cell, but is introduced into a cell by one or more genetic, biochemical or other methods. An exogenous molecule can be, among other things, a small molecule, such as is generated by a combinatorial chemistry process, or a macromolecule such as a protein, nucleic acid, carbohydrate, lipid, glycoprotein or lipoprotien. For example, an exogenous nucleic acid can comprise an infecting viral genome, a plasmid or episome introduced into a cell, or a chromosome that is not normally present in the cell. Methods for the introduction of exogenous nucleic acids into cells are known to those of skill in the art and exemplary methods are described *infra.* An exogenous molecule can comprise, for example, a functioning version of a malfunctioning endogenous molecule or a malfunctioning version of a normally-functioning endogenous molecule.

Modulation of expression of a gene refers to a change in the activity of a gene. Modulation of expression can include, but is not limited to, gene activation and gene repression.

Gene activation is any process which results in an increase in production of a gene product. A gene product can be either RNA (including, but not limited to, mRNA, rRNA, tRNA, and structural RNA) or protein. Accordingly, gene activation includes those processes which increase transcription of a gene and/or translation of a mRNA. Examples of gene activation processes which increase transcription include, but are not limited to, those which facilitate formation of a transcription initiation complex, those which increase transcription initiation rate, those which increase transcription elongation rate, those which increase processivity of transcription and those which relieve transcriptional repression (by, for example, blocking the binding of a transcriptional repressor). Examples of gene activation processes which increase translation include those which increase translational initiation, those which increase translational elongation and those which increase mRNA stability.

Gene repression is any process which results in a decrease in production of a gene product. A gene product can be either RNA (including, but not limited to, mRNA, rRNA, tRNA, and structural RNA) or protein. Accordingly, gene repression includes those processes which decrease transcription of a gene and/or translation of a mRNA. Examples of gene repression processes which decrease transcription include, but are not limited to, those which inhibit formation of a transcription initiation complex, those which decrease transcription initiation rate, those which decrease transcription elongation rate, those which decrease processivity of transcription and those which antagonize transcriptional activation (by, for example, blocking the binding of a transcriptional activator). Examples of gene repression processes which decrease translation include those which decrease translational initiation, those which decrease translational elongation and those which decrease mRNA stability. Transcriptional repression includes both reversible and irreversible inactivation of gene transcription.

Eucaryotic cells include, but are not limited to, fungal cells (such as yeast), plant cells, animal cells, mammalian cells and human cells.

A region of interest is any region of cellular chromatin, such as, for example, a gene or a non-coding sequence within or adjacent to a gene, in which it is desirable to bind an exogenous molecule. A region of interest can be present in a chromosome, an episome, an organellar genome (*e.g*., mitochondrial, chloroplast), or an infecting viral genome, for example. A region of interest can be within the coding region of a gene, within transcribed non-coding regions such as, for example, leader sequences, trailer sequences or introns, or within non-transcribed regions, either upstream or downstream of the coding region.

### Accessible regions

An accessible region in cellular chromatin is generally one that does not have a typical nucleosomal structure. As such, an accessible region can be identified and localized by, for example, the use of chemicals and/or enzymes that probe chromatin structure. Accessible regions will, in general, have an altered reactivity to a probe, compared to bulk chromatin. An accessible region may be sensitive to the probe, compared to bulk chromatin, or it may have a pattern of sensitivity that is different from the pattern of sensitivity exhibited by bulk chromatin. Accessible regions can be identified by any method known to those of skill in the art for probing chromatin structure.

In one embodiment, an enzymatic probe of chromatin structure is used to identify an accessible region. In a preferred embodiment, the enzymatic probe is DNase I (pancreatic deoxyribonuclease). Regions of cellular chromatin that exhibit enhanced sensitivity to digestion by DNase I, compared to bulk chromatin (*i.e*., DNase-hypersensitive sites) are more likely to have a structure that is favorable to the binding of an exogenous molecule, since the nucleosomal structure of bulk chromatin is generally less conducive to binding of an exogenous molecule. Furthermore, DNase-hypersensitive regions of chromatin often contain DNA sequences involved in the regulation of gene expression. Thus, binding of an exogenous molecule to a DNase-hypersensitive chromatin region is more likely to have an effect on gene regulation.

In a separate embodiment, micrococcal nuclease (MNase) is used as a probe of chromatin structure to identify an accessible region. MNase preferentially digests the linker DNA present between nucleosomes, compared to bulk chromatin. It is likely that such linker DNA sequences are more apt to be bound by an exogenous molecule that are sequences present in nucleosomal DNA, which is wrapped around a histone octamer.

Additional enzymatic probes of chromatin structure include, but are not limited to, exonuclease III, S1 nuclease, mung bean nuclease, DNA methyltransferases and restriction endonucleases. In addition, the method described by van Steensel et al. (2000) Nature Biotechnology 18:424-428 can be used to identify an accessible region.

Chemical probes of chromatin structure, useful in the identification of accessible regions, include, but are not limited to, hydroxyl radicals, methidiumpropyl-EDTA.Fe(II) (MPE) and crosslinkers such as psoralen. *See,* for example, Tullius et al. (1987) Meth. Enzymology, Vol. 155, (J. Ableson & M. Simon, eds.) Academic Press, San Diego, pp. 537-558; Cartwright et al. (1983) Pro. Natl. Acad. Sci. USA 80:3213-3217; Hertzberg et al. (1984) Biochemistry 23:3934-3945; and Wellinger et al. in Methods in Molecular Biology, Vol. 119 (P. Becker, ed.) Humana Press, Totowa, NJ, pp. 161-173.

Localization of sequences that have altered reactivity to enzymatic and chemical probes, compared to bulk chromatin, is accomplished by methods known to those of skill in the art. *See*, for example, Wu in Methods in Enzymology, Vol. 170, (J. Abelson & M. Simon, eds.) Academic Press, San Diego, pp. 269-289; and Cockerill in Methods in Molecular Biology, Vol. 130 (M.J. Tymms, ed.), Humana Press, Totowa NJ, 2000, pp. 29-46. In one embodiment, the technique of indirect end-labeling is used. In this method, cellular chromatin (for example, in the form of isolated nuclei) is first exposed to the action of an enzymatic or chemical probe of chromatin structure, then deproteinized and digested with a restriction enzyme that will generate a restriction fragment which includes the region of interest. Following digestion, DNA fragments are separated by gel electrophoresis and blotted onto a membrane. The membrane is then hybridized with a labeled hybridization probe complementary to a short region at one end of the restriction fragment containing the region of interest. In the absence of an accessible region, the hybridization probe will identify the full-length restriction fragment. However, if an accessible region is present within the sequences defined by the restriction fragment, the hybridization probe will identify one or more DNA species that are shorter than the restriction fragment. The size of these additional DNA species corresponds to the distance between the accessible region and the end of the restriction fragment to which the hybridization probe is complementary. *See,* for example, Figure 1A.

### Target sites

Once an accessible region is identified, a search for a target site can be conducted within the nucleotide sequence of the accessible region. For exogenous molecules which do not have binding specificity, or which exhibit a relaxed or promiscuous specificity, it may not be necessary to identify a target site. Exogenous molecules such as proteins and, in particular, transcription factors, often have a preferred target site. In these cases, the nucleotide sequence of the accessible region can be searched for the presence of the preferred target site. Target sites for various transcription factors are known. *See,* for example, Wingender et al. (1997) Nucleic Acids Res. 25:265-268 and the TRANSFAC Transcription Factor database at http://transfac.gbf.de/TRANSFAC/, accessed on April 13, 2000. In general, target sites for newly-discovered transcription factors, as well as other types of exogenous molecule, can be determined by methods that are well-known to those of skill in the art such as, for example, electrophoretic mobility shift assay, exonuclease protection, DNase footprinting, chemical footprinting and/or direct nucleotide sequence determination of a binding site. *See,* for example, Ausubel *et al., supra,* Chapter 12.

A target site is a nucleic acid sequence that defines a portion of a nucleic acid to which a binding molecule will bind, provided sufficient conditions for binding exist. Although binding of a molecule to its target site will generally occur in a naked nucleic acid molecule, a binding molecule may be incapable of binding to its target site in cellular chromatin, as a result of some aspect of the structure of the chromatin in which the target site is located. Alternatively, factors in addition to a target site may be required for binding of a molecule to a target site. For instance, binding of a molecule to a polynucleotide comprising a target site may require (or be strengthened by) contact with both specific amino acid sequences and specific polynucleotide sequences.

Accordingly, a binding site in cellular chromatin is a region at which a particular molecule, for example a protein, will bind to a target site in the chromatin. A binding site will generally comprise a target site, but not every target site will constitute a binding site in cellular chromatin. For example, a target site may be occluded by one or more chromosomal components, such as histones or nonhistone proteins, or might be rendered inaccessible to its binding molecule because of nucleosomal or higher-order chromatin structure. On the other hand, the presence of one or more chromosomal proteins may be required, in addition to a target site, to define a binding site.

### Exogenous molecules

An exogenous molecule, with respect to a particular cell, is any molecule that is not normally present in the cell. "Normal presence in the cell" is determined with respect to the particular developmental stage and environmental conditions of the cell. By contrast, an endogenous molecule is one that is normally present in a particular cell at a particular developmental stage under particular environmental conditions. Thus, for example, a molecule that is present only during embryonic development of muscle is an exogenous molecule with respect to an adult muscle cell. Similarly, a molecule induced by heat shock is an exogenous molecule with respect to a non-heat-shocked cell.

An exogenous molecule can be the same type of molecule as an endogenous molecule, *e.g*., protein or nucleic acid, providing it has a sequence that is different from an endogenous molecule. An exogenous molecule can be introduced into a cell by any method known to one of skill in the art including, but not limited to, lipid-mediated transfer (including neutral and cationic lipids), electroporation, direct injection, particle bombardment, calcium phosphate co-precipitation, DEAE-dextran-mediated transfer and viral vector-mediated transfer.

Exogenous molecules include, but are not limited to, macromolecules such as proteins, nucleic acids, lipids and polysaccharides, as well as small molecules such as those that might be generated by processes of drug discovery or combinatorial chemistry. *See*, for example, WO 93/06121; WO 94/08051; WO 95/12608; WO 95/30642; and WO 95/35503. Nucleic acids include RNA and DNA; can be single- or double-stranded; can be linear, branched or circular; and can be of any length. Nucleic acids include those capable of forming duplexes and those capable of forming triplex structures with double-stranded DNA. *See,* for example, U.S. Patent No. 5,422,251 and U.S. Patent No. 5,176,996. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

In a preferred embodiment, an exogenous molecule is a zinc finger DNA-binding protein (ZFP). Certain ZFPs, their properties and their binding sequences are known in the art, as described *supra.* Furthermore, it is possible, for any particular nucleotide sequence, to design and/or select one or more ZFPs capable of binding to that sequence and to characterize the affinity and specificity of binding. *See,* for example, U.S. Patent No. 5,789,538; U.S. Patent No. 6007,408; U.S. Patent No. 6013,453; PCT WO 95/19431; PCT WO 98/54311 co-owned WO 00/42219 and references cited therein. Certain sequences, such as those that are G-rich, are preferred as ZFP binding sites. Since a three-finger ZFP generally binds to a 9- or 10-nucleotide target site, in a preferred embodiment, an accessible region, present within a region of interest in cellular chromatin, is searched for one or more G-rich sequences of 9-10 nucleotides and, for each sequence so detected, a ZFP can be designed to bind those sequences. In addition, two three finger modules can be joined, via an appropriate linker domain, to form a six-finger protein capable of recognizing an 18-20 nucleotide target site. *See*, for example, PCT/US99/04441.

The aforementioned categories of exogenous molecules include analogues and modified variants. For example, nucleic acids can include modified bases, sugars and/or internucleotide linkages. Nucleic acid analogues include polyamide (peptide) nucleic acids and chimeric molecules comprising PNA and/or DNA and/or RNA. *See,* for example, Nielsen et al. (1991) Science 254:1497-1500; Uhlmann (1998) Biol. Chem. 379:1045-1052. DNA/RNA hybrids and DNA/RNA chimeras are also included. Protein analogues include those comprising modifications such as, for example, acetylation, phosphorylation and myristylation, as well as those containing non-naturally-occuring amino acids, amino acid variants and/or non-peptide inter-amino acid linkages.

In certain embodiments, an exogenous molecule can be responsible for the production of one or more additional exogenous molecules in a cell. For example, an exogenous molecule can be a transcription factor that induces the expression of genes that are not normally expressed in the cell. These newly-expressed genes may in turn, be responsible for the production of yet additional exogenous molecules in the cell. For example, induction of enzymes involved in intermediary metabolism would lead to the presence of new metabolic intermediates in the cell. Alternatively, an exogenous nucleic acid can be responsible for the production of an exogenous protein such as, for example, a transcription factor. Exogenous nucleic acids can be either integrated or episomal, and can be either stably or transiently present in the cell.

Exogenous molecules include variants and analogues of molecules normally present in the cell, no matter how such a variant or analogue may be obtained. Variants and analogues of, for example, a protein, can comprise insertion(s), deletion(s), and/or rearrangement(s) of amino acids or inclusion of non-naturally-occurring and/or modified amino acids. Such variants and analogues of a protein can be obtained, for example, by design and synthesis of a protein variant or analogue; by chemical, enzymatic or other modification of a protein; or by mutagenesis, either directed or random, of a nucleic acid encoding a protein. Appropriate selection methods, as are known in the art, can be used to select a particular variant or analogue from among a population of proteins or nucleic acids. *See,* for example, U.S. Patent No. 5,789,538; Greisman et al. (1997) Science 275:657-661; U.S. Patent No. 6007,408; U.S. Patent No. 6,013,453; PCT WO 91/18980; PCT WO 95/19431; PCT WO 98/54311 co-owned WO 00/42219 and references cited therein. Variants and/or analogues of a small molecule can be obtained by, for example, substitution of various functional groups on a molecular scaffold.

### Tests for binding

In certain embodiments, interaction of an exogenous molecule with a binding site can be confirmed by one of a number of tests. Any method known to one of skill in the art, for detection of binding to chromatin, is applicable. One such test is *in vivo* footprinting, in which the accessibility of particular nucleotides to chemical probes is determined. Changes in accessibility of particular sequences in the presence of an exogenous molecule are indicative of binding of the exogenous molecule to those sequences. *See,* for example, Wassarman and Wolffe, eds., Methods in Enzymology, Volume 304, Academic Press, San Diego, 1999.

In a preferred embodiment, sequence-specific binding of an exogenous molecule to chromatin is assayed by chromatin immunoprecipitation (ChIP). Briefly, this technique involves the use of a specific antibody to immunoprecipitate chromatin complexes comprising the corresponding antigen, and examination of the nucleotide sequences present in the immunoprecipitate. Immunoprecipitation of a particular sequence by the antibody is indicative of interaction of the antigen with that sequence. *See,* for example, O'Neill et al. in Methods in Enzymology, Vol. 274, Academic Press, San Diego, 1999, pp. 189-197; Kuo et al. (1999) Method 19:425-433; and Ausubel *et al., supra,* Chapter 21.

In one embodiment, the chromatin immunoprecipitation technique is applied as follows. An exogenous molecule is introduced into a cell and, after a period of time sufficient for binding of the exogenous molecule to its binding site has elapsed, cells are treated with an agent that crosslinks an exogenous molecule to chromatin if that molecule is stably bound. If the exogenous molecule is a protein, it can be crosslinked to chromatin by, for example, formaldehyde treatment or ultraviolet irradiation. Subsequent to crosslinking, cellular nucleic acid is isolated, sheared and incubated in the presence of an antibody directed against the exogenous molecule. Antibody-antigen complexes are precipitated, crosslinks are reversed (for example, formaldehyde-induced DNA-protein crosslinks can be reversed by heating) and the sequence content of the immunoprecipitated DNA is tested for the presence of a specific sequence, for example, the target site of the exogenous molecule.

In a preferred embodiment, the immunoprecipitated DNA is tested for the presence of specific sequences by a sensitive hydrolyzable probe assay allowing real-time detection of an amplification product, known colloquially as the Taqman^{®} assay. *See* U.S. Patent No. 5,210,015; Livak et al. (1995) PCR Meth. App. 4:357-362 and Heid et al. (1996) Genome Res. 6:986-994. Briefly, an amplification reaction (*e.g.,* PCR) is conducted using a probe designed to hybridize to a target sequence flanked by two amplification primers. The probe is labeled with a fluorophore and a fluorescence quencher such that, when not hybridized to its target sequence, the probe does not emit detectable fluorescence. Upon hybridization of the probe to its target and hydrolysis of the probe by the polymerase used for amplification, the fluorophore is released from the vicinity of the quencher, and fluorescence increases in proportion to the concentration of amplification product. In this assay, the presence of increased levels of an amplification product corresponding to the binding site for the exogenous molecule, compared to levels of amplification product specific to a control genomic sequence, is indicative of binding of an exogenous molecule to its binding site in cellular chromatin.

Additional methods for detecting binding of an exogenous molecule to chromatin include, but are not limited to, microscopy (*e.g.,* scanning probe microscopy), fluorescence *in situ* hybridization (FISH) and fusion of a DNA methylase domain to the exogenous molecule, in which case sequences to which the exogenous molecule is bound become methylated and can be identified, for example, by comparing their sensitivity to methylation-sensitive and methylation-dependent restriction enzymes or by using antibodies to methylated DNA. *See,* for example, van Steensel *et al.*, *supra.*

### Applications

The methods disclosed herein are useful in a variety of applications and provide advantages over existing methods. These include therapeutic methods in which an exogenous molecule is administered to a subject and used to modulate expression of a target gene within the subject. *See,* for example, co-pending PCT/US00/00409. Modulation of gene expression can be in the form of repression as, for example, when the target gene resides in a pathological infecting microorganism or in an endogenous gene of the subject, such as an oncogene or a viral receptor, that contributes to a disease state. Alternatively, modulation can be in the form of activation, if activation of a gene (*e.g.,* a tumor suppressor gene) can ameliorate a disease state. For such applications, an exogenous molecule can be formulated with a pharmaceutically acceptable carrier, as is known to those of skill in the art. *See,* for example, Remington's Pharmaceutical Sciences, 17th ed., 1985; and co-owned PCT/US00/00388.

Binding of an exogenous molecule to a binding site in cellular chromatin can be used for detection of a particular sequence as in, for example, diagnostic applications. Methods for detection of a target sequence using, for example, a ZFP are described in co-owned PCT/US00/00388. For example, an exogenous molecule, such as a sequence-specific DNA binding protein, can be used to detect variant alleles associated with a disease or with a particular phenotype in patient samples and to detect the presence of pathological microorganisms in clinical samples. In one embodiment, a variant allele comprises a single-nucleotide polymorphism (SNP). In a non-mutually exclusive embodiment, the sequence-specific DNA binding protein is a ZFP. Exogenous molecules can also be used to quantify copy number of a gene in a sample. For example, detection of the loss of one copy of a p53 gene in a clinical sample is an indicator of susceptibility to cancer.

Current methodologies for determination of gene function rely primarily upon either overexpression of a gene or removal of a gene from its natural biological setting (*i.e.,* gene knock-out), followed by observation of effects. The phenotypic effects observed can give indications of the role of the gene in the biological system. However, graded levels of gene expression are difficult to obtain using these methods; furthermore it is impossible to use gene removal (*i.e*., knock-out) technology to determine adult function for a gene required in early development.

The use of assays involving the binding of exogenous molecules to cellular chromatin can overcome these difficulties. For example, if an exogenous molecule is a protein, an exogenous gene encoding the protein can be introduced into a cell and placed under small molecule control. By controlling the level of expression of an exogenous molecule in this way, it is possible to control the expression levels of a gene regulated by the exogenous molecule, thereby allowing one to determine what level of expression of a gene (*i.e.*, what degree of either repression or stimulation of expression) is required to achieve a given phenotypic or biochemical effect.

This approach has particular value for drug development. By placing expression of an exogenous molecule under small molecule control in, for example, a transgenic animal, problems of embryonic lethality and developmental compensation can be avoided by activating or inhibiting gene expression at later stages in development and observing effects in the adult animal. For example, transgenic mice having a target gene(s) regulated by a ZFP can be produced by integration of a nucleic acid encoding the ZFP at any site in *trans* to the target gene. Accordingly, homologous recombination is not required for integration of the nucleic acid. Further, because an integrated ZFP-encoding gene is *trans*-dominant, only a single chromosomal copy is required and functional knock-out animals, if desired, can be produced without backcrossing.

Thus, methods of binding of an exogenous molecule to cellular chromatin, as disclosed herein, can be used in assays to determine gene function and to determine changes in phenotype resulting from specific modulation of gene expression.

Identification of a binding site for an exogenous molecule, within a region of interest in cellular chromatin, facilitates the formation of a complex between the exogenous molecule and its binding site after the exogenous molecule has been introduced into the cell. Accordingly, complexes between an exogenous molecule and its binding site in cellular chromatin are provided. Such complexes are useful in the modulation of gene expression by either activation or repression of transcription (depending upon the action of the exogenous molecule). The complexes can be transient or stable and can be formed on chromosomal, episomal, or any other type of chromatin.

The following examples are presented as illustrative of, but not limiting, the claimed subject matter.

### EXAMPLES

### Example 1: Cell Growth and isolation of nuclei for studies of nuclease hypersensitivity

Transformed human embryonic kidney 293 cells were grown in DMEM + 10% fetal calf serum, supplemented with penicillin and streptomycin, in a 37°C incubator at 5% CO₂. Typically, two 255 cm² plates of cells were used in an experiment. When the cells reached greater than 90% confluence (~2.5 x 10⁷ cells per plate), medium was removed and the cells were rinsed twice with 5 ml of ice-cold PBS (Gibco/Life Technologies, Gaithersburg, MD). Cells were then scraped from the plates in 5 ml of ice-cold PBS and combined in a 50 ml conical centrifuge tube. The plates were then washed with 10 ml of ice-cold PBS and the washes were added to the tube. Nuclei were pelleted by centrifugation (1400 rpm for 5 min) and the supernatant was removed. The pellet was mixed by vortexing and, while vortexing, 20 ml of lysis buffer (10 mM Tris pH 7.5, 1.5 mM MgCl₂, 10 mM KCl, 0.5% IGEPAL CA-630 (Sigma), 1 mM phenylmethylsulfonyl fluoride, 1 mM dithiothreitol) was added. The cell pellet was resuspended in lysis buffer by pipetting and the tube was centrifuged at 1400 rpm for 5 min. The supernatant was removed and the pellet was resuspended in 20 ml of lysis buffer and centrifuged as before. The final pellet was resuspended in 1.5 ml dilution buffer (15 mM Tris pH 7.5, 60 mM KCl, 15 mM NaCl, 5 mM MgCl₂, 0.1 mM dithiothreitol, 10% glycerol), nuclei were counted in a microscope and the solution was adjusted so that a concentration of approximately 10⁷ nuclei per ml was obtained.

### Example 2: DNase treatment of nuclei

Nuclei, at a concentration of 10⁷ per ml in dilution buffer, were digested with different concentrations of DNase I. DNase I dilutions were prepared by diluting deoxyribonuclease I (Worthington, Freehold, NJ) in dilution buffer (see previous example) supplemented with 0.4 mM CaCl₂. To 100 µl of resuspended nuclei was added 25 µl of a DNase I dilution to give final DNase I concentrations ranging from 0.07 Units/ml to 486 Units/ml in three-fold concentration increments. Digestions were conducted at room temperature for 5 min. Digestion reactions were then stopped by addition of 125 µl of Buffer AL (Qiagen DNeasy™ Tissue Kit) and 12.5 µl of a 20 mg/ml solution of Proteinase K (Qiagen DNeasy™ Tissue Kit), followed by incubation at 70°C for 10 min. Digested DNA was purified using the DNeasy™ Tissue Kit (Qiagen, Valencia, CA) according to the manufacturer's instructions.

Purified DNase-treated DNA was digested with restriction enzyme at 37°C overnight with 40 Units of restriction enzyme in the presence of 0.4 mg/ml RNase A. For the analysis shown in Figure 1, an Xba I digestion was conducted. After digestion, DNA was ethanol-precipitated from 0.3 M sodium acetate.

### Example 3: Micrococcal nuclease treatment of nuclei

Treatment of nuclei, obtained as described *supra,* with micrococcal nuclease is conducted as described by Livingstone-Zatchej et al. in Methods in Molecular Biology, Vol. 119, Humana Press, Totowa, NJ, pp. 363-378.

### Example 4: Treatment of nuclei with a chemical probe

Nuclei are treated with MPE using the following procedure adapted from Cartwright *et al., supra.* A freshly-diluted stock of 0.4 M H₂O₂ is prepared by making a 25-fold dilution of a 30% stock solution. A freshly-prepared stock of 0.5 M ferrous ammonium sulfate is diluted 400-fold in water. A solution of methidiumpropyl EDTA (MPE) is prepared by adding 30 µl of 5 mM MPE to 90µl of water. To this MPE solution is added 120 µl of the ferrous ammonium sulfate dilution and 2.5 µl of 1 M dithiothreitol (DTT, freshly prepared from powder). To a suspension of nuclei, obtained as described *supra,* are added, in sequence: 3.5 µl of 0.4 M H₂O₂ and 37.5 µl of the MPE/ferrous ammonium sulfate/DTT mixture. The reaction is terminated after an appropriate time period (determined empirically) by addition of 40 µl of 50 mM bathophenanthroline disulfonate, 0.1 ml of 2.5% sodium dodecyl sulfate/50 mM EDTA/50 mM Tris-Cl, pH 7.5 and 10 µl of Proteinase K (10-14 mg/ml). Digestion is conducted at 37°C for at least 8 hours and the mixture is then extracted twice with phenol/chloroform and once with chloroform. Nucleic acids are precipitated from the aqueous phase by addition of sodium acetate to 0.3 M and 0.7 volume of isopropyl alcohol, incubation on ice for at least 2 hr, and centrifugation. The pellet is washed with 70% ethanol, dried, resuspended in 10 mM Tris-Cl, pH 8 and treated with RNase A (approximately 0.1 mg/ml) for 15 min at 37°C.

### Example 5: Blotting and hybridization

Pellets of precipitated, digested DNA obtained according to Examples 2, 3 or 4 were resuspended in 22 µl of loading buffer containing glycerol and tracking dyes ("Gel loading solution," Sigma Chemical Corp., St. Louis, MO) and incubated at 55°C for 3-4 hours. Twenty microliters of resuspended sample was loaded onto a 1% agarose gel containing 1X TAE buffer and 0.5 µg/ml ethidium bromide, and electrophoresis was conducted at 22 Volts for 16 hours in Tris-acetate-EDTA buffer. After electrophoresis, the gel was treated with alkali, neutralized, blotted onto a Nytran membrane (Schleicher & Schuell, Keene, NH), and the blotted DNA was crosslinked to the membrane by ultraviolet irradiation.

Probes were labeled by random priming, using the Prime-It Random Primer Labeling Kit (Stratagene, La Jolla, CA) according to the manufacturer's instructions. In a typical labeling reaction, 25-50 ng of DNA template was used in a final volume of 50 µl. A specific activity of 10⁹ cpm/µg was typically obtained. Labeled probes were purified on a NucTrap probe column (Stratagene #400702, La Jolla, CA).

The membrane was placed in a hybridization bottle and pre-hybridized in Rapid Hybridization Buffer (Amersham, Arlington Heights, IL) at 65°C for 15 min. Probe (a 0.1 kb XbaI-KpnI fragment, see Figure 1A) was added (approximately 0.03 µg containing approximately 3.3 x 10⁷ cpm) and hybridization was conducted at 65°C for 2 hours. Following hybridization, the membrane was washed once at 65°C for 10 min. with 2X SSC + 0.1% SDS, and twice at 65°C for 10 min. with 0.1X SSC + 0.1% SDS. The membrane was then dried and analyzed either by autoradiography or with a phosphorimager.

Results are shown in Figure 1B for analysis of DNase hypersensitivity within a 10.5 kb region comprising the human erythropoietin (EPO) gene in 293 cells. Increasing DNase concentration resulted in the generation of two new DNA fragments, of 3.3 and 3.9 kb, indicating the presence of two DNase hypersensitive sites located downstream of the EPO coding region. See Figure 1A.

### Example 6: Reporter cells for chromatin immunoprecipitation analysis

A transformed human embryonic kidney cell line (293 cells) containing a stably integrated luciferase gene was used as a reporter cell line. The reporter construct, pVFR3-4X, was a pGL3 vector (Promega, Madison, WI) containing a firefly luciferase gene under the control of the SV40 promoter, into which four tandem copies of a target site for the VEGF 3a/1 ZFP were inserted upstream of the promoter, between the Mlu I and Bgl II sites. See Example 8 for the sequences of VEGF 3a/1 and its target site.

Integration of the reporter construct into the genome of 293 cells and selection of integrants was accomplished as follows. 10 µg of the reporter plasmid pVFR3-4X and 1 µg of pSV2Neo were co-transfected into HEK293 cells by Lipofectamine (Gibco-Life Technologies)-mediated transfection. Forty-eight hours post-transfection, the cells were trypsinized and plated at a 1:500 split ratio into 15-cm dishes and placed under G418 selection (500 mg/ml). Single clones were isolated after 14 days of selection. Selected clones were analyzed for basal luciferase activity, using a PE/Tropix Dual-Light^{®} assay system. Preparation of cell extracts and measurement of luciferase activity were performed according to the manufacturer's instructions. Clone 42 was selected, expanded and used for the examples described below.

Cells were grown in 10 cm dishes in DMEM supplemented with glutamine, penicillin, streptomycin and 10% fetal bovine serum. Cells were cultured at 37°C in 5% CO₂ and, when near confluence (approximately 0.5-1 X 10⁷ cells per dish), were collected for analysis.

### Example 7: Accessible regions in the human Vascular Endothelial Growth Factor-A (VEGF-A) gene

The presence of DNase hypersensitive sites in the upstream region of the human VEGF gene (Tischer et al. (1991) J. Biol. Chem.266:11,947-11,954) was examined by DNase digestion of nuclei from human 293 cells, followed by indirect end labeling, as described in Examples 1, 2 and 5 *supra.* Representative results are shown in Figure 2, in which the presence of two accessible regions, centered around +1 (-100 to +100) and -550 (-600 to -500), with respect to the transcriptional startsite, were identified. *See* also Liu et al. (2001) J. Biol. Chem. 276:11,323-11,334.

### Example 8: ZFP-encoding plasmids

Plasmids were constructed to encode transcriptional effector proteins containing zinc finger domains designed to recognize target sites surrounding the transcriptional initiation site of the human vascular endothelial growth factor (VEGF) gene; i.e. within the +1 accessible region described in Example 7. The target site has the sequence 5'-GGGGAGGATCGCGGAGGCTT-3' (SEQ ID NO: 1), where the underlined T residue represents the major transcriptional startsite for the VEGF gene. A binding domain containing six zinc fingers, named VEGF 3a/1, was designed to bind to this 20-nucleotide target sequence. A three-finger zinc finger domain, VEGF 1 was designed to bind to the upstream 10-nucleotides of this target site having the sequence 5'-GGGGAGGATC-3' (SEQ ID NO: 2). A control six-finger domain, GATA 15.5, which was designed to bind the sequence 5'-GAGTGTGTGAACTGCGGGGCAA-3' (SEQ ID NO: 3), was also used. These zinc finger domains were encoded as fusion proteins in the NVF vector, as described below.

The zinc finger domains were constructed in a SP1 backbone. The sequences of the recognition helices, from position-1 to position +6, of VEGF 3a/1, VEGF 1 and GATA 15.5 are shown in Table 1.

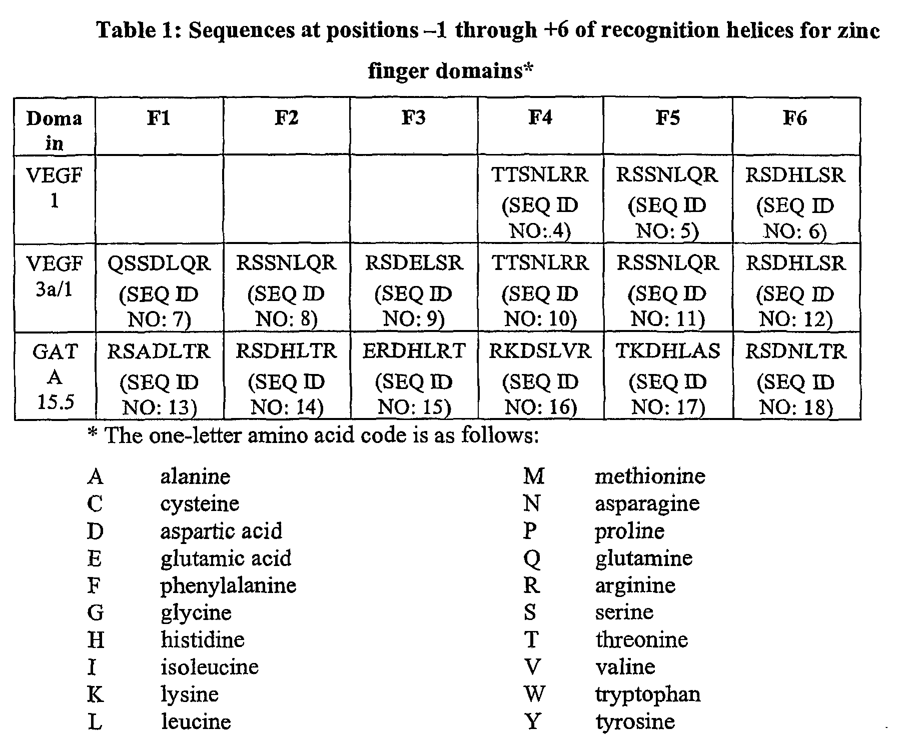

The control plasmid NVF contains sequences encoding a fusion protein comprising a nuclear localization signal, a VP16 activation domain and a FLAG epitope (in amino-to-carboxy order in the encoded protein) in a pcDNA3.1 (+) (Invitrogen) plasmid backbone. Transcription of the mRNA encoding the fusion protein is under the control of a CMV promoter, and translational initiation is specified by a Kozak sequence. Kozak (1991) J. Biol. Chem. 266:19867-19870. Transcriptional termination is specified by a bovine growth hormone polyadenylation sequence. The NVF plasmid does not contain sequences encoding a zinc finger domain. This plasmid was used for insertion of sequences encoding the zinc finger domains shown in Table 1, and as a control for experiments in which exogenous ZFPs were introduced into cells.

The nuclear localization sequence (NLS) encoded in the NVF plasmid is from the SV40 large T antigen and encodes the amino acid sequence Pro-Lys-Lys-Lys-Arg-Lys-Val. Kalderon et al. (1984) Cell 39:499-509. The VP16 activation domain contains amino acids 413 to 490 of the VP16 protein sequence. Hagmann et al. (1997) J. Virology 71:5952-5962. The FLAG epitope (Kodak) is included to allow specific detection of plasmid-encoded proteins. The vector also includes markers for ampicillin and neomycin resistance, for selection in bacterial and mammalian cells, respectively. A map of the NVF plasmid is shown in Figure 3.

For construction of plasmids including a zinc finger binding domain, ZFP-encoding sequences were inserted into the NVF plasmid between the NLS and the VP16-encoding domains. The zinc finger domains contained designed recognition helices, as shown in Table 1, in a SP1 backbone.

Further details on the synthesis of these constructs, purification of the encoded proteins, and tests for binding affmity and specificity are provided in co-owned PCT/US00/00409.

### Example 9: Transfection of ZFP-encoding plasmids into reporter cell lines

Reporter cells (see Example 6) were transfected with ZFP-encoding or control plasmids, as described in Example 8. Twenty-four hours prior to transfection, cells were plated in 10 cm dishes at a density of 2.5 x 10⁶ per plate. For each transfection, 10 µg of plasmid DNA was diluted in 2.5 ml Opti-MEM (Life Technologies), and 50 µl of Lipofectamine 2000 was diluted in 2.5 ml Opti-MEM. The diluted DNA and lipid were mixed and incubated for 20 minutes at room temperature. Medium was then removed from the cells and replaced with the lipid/DNA mixture. Cells were incubated at 37°C for 3 hours in a CO₂ incubator, then 10 ml of DMEM+10% FBS was added. Two days after transfection, medium was removed from the transfected cells and cells were processed for chromatin immunoprecipitation as described in Example 11.

### Example 10: Measurement of luciferase activity in transfected cells

Reporter cells were harvested approximately 48 hours after transfection with ZFP-encoding or control plasmids, and approximately 1.5-2 x 10⁶ cells were used in an assay. Luciferase activity encoded by the integrated reporter gene was measured using a PE/Tropix Dual-Light^{®} assay system. Preparation of cell extracts and measurement of luciferase activity were performed according to the manufacturer's instructions.

### Example 11: Binding of exogenous ZFPs to the human vascular endothelial growth factor (VEGF) gene assayed by chromatin immunoprecipitation

### Crosslinking

A 1% (v/v) solution of formaldehyde was prepared by adding 14 ml of 37% aqueous formaldehyde to 500 ml of PBS (Sigma). Cells were transfected and cultured as described in Example 9. Two days after the cells were transfected, medium was aspirated and 10 ml of a 1% (v/v) solution of formaldehyde in PBS was added. Plates were incubated for 15 min at room temperature, with shaking every 5 min. The formaldehyde solution was then removed and the plates were washed twice with 10 ml of 50 mM Tris-Cl (pH 7.5), 150 mM NaCl.

### Lysis and sonication

Cells were lysed by addition of 0.5 ml per plate of WCLB (50 mM HEPES (pH 7.6), 150 mM NaCl, 0.1% (v/v) NP-40, 5 mM EDTA) containing protease inhibitors (Roche Diagnostics #1836153, one tablet per 10 ml) plus 0.1% (w/v) sodium dodecyl sulfate, followed by incubation on ice for 10 min. The lysate was removed by scraping the plate and was transferred to a microfuge tube. The lysate was sonicated, using a VirSonic sonicator (Virtis Instruments) equipped with a microtip, at a power setting of 4. Sonication was conducted on ice in bursts of 5 sec, at 5 sec. intervals, for a total of 5 min. The majority of the chromatin fragments generated using these sonication conditions ranged in size from 100 to 200 nucleotide pairs. These conditions can be varied, as long as the appropriate size distribution is obtained.

Following sonication, 1 ml of WCLB was added, and the sonicated lysate was subjected to centrifugation at top speed in a microfuge (approx. 15,000 rpm, 13,000 xg) for 10 min at 4°C. The supernatant was collected, and divided into three portions: a sample for immunoprecipitation (0.7 ml), an input control (0.1 ml) and a no-antibody control (0.7 ml).

### Immunoprecipitation

The sample for immunoprecipitation was treated as follows. Anti-FLAG M2 antibody (Sigma, St. Louis, MO, Catalogue #F3165) was added to a final concentration of 1 µg/ml, and the sample was incubated, with shaking, at 4°C for 2 hours. (Antibodies directed against other portions of the protein can also be used. For example, anti-VP16 antibodies have also been used.) Then, 30 µl of a slurry of Protein G beads (Amersham/Pharmacia Biotech, Piscataway, NJ), pre-equilibrated with WCLB, was added and incubation at 4°C was continued overnight.

After overnight incubation, the sample was centrifuged in a microfuge at 2,000 rpm for 5 min, and the supernatant was removed. The protein G beads were washed twice, for 3 min each time, with WCLB, twice with WCLB containing 1M NaCl, and once with TE (Sigma T-9285), then resuspended in 0.1 ml of TE. Twenty micrograms of RNase A (Sigma R-6513) was added, and the sample was incubated at 37°C for 30 min. The beads were sedimented, and the supernatant was removed.

Immunoprecipitated material was eluted from the Protein G beads by adding 0.1 ml of 50 mM Tris-Cl (pH 8.0), 10 mM EDTA, 1% (w/v) sodium dodecyl sulfate and incubating at 65°C for 15 min. The supernatant was collected and a second elution, identical to the first, was conducted. The eluates were combined, and 0.2 ml of TE was added to the combined eluates, to give a final volume of 0.4 ml. This solution was then incubated at 65°C for at least 5 hours (not to exceed an overnight incubation), during which time formaldehyde-induced crosslinks were reversed.

Following reversal of crosslinks, Proteinase K (Sigma P-2308) was added to 0.4 mg/ml and the mixture was incubated at 50°C for 2 hours. At the conclusion of the incubation, 20 µg of glycogen and 20 µl of 5 M NaCl were added, and the mixture was extracted once with phenol/chloroform/isoamyl alcohol (25:24:1, v/v) and once with chloroform/isoamyl alcohol (24:1, v/v). The aqueous phase was retained, and nucleic acid was precipitated after addition of 2.5 volumes of ethanol, followed by centrifugation in a microcentrifuge at maximum speed for 10 min. The pellet was washed with 70% ethanol, dried and resuspended in 50 µl of TE.

### Analysis of immunoprecipitated material by real-time PCR

The presence of particular DNA sequences in immunoprecipitates was tested using a PCR-based, hydrolyzable probe assay known as TaqMan^{®}. Briefly, a region of interest is amplified by PCR using two oligonucleotide primers: a forward primer and a reverse primer. A third oligonucleotide, known as the probe oligonucleotide, is designed to hybridize within the region being amplified. The probe oligonucleotide comprises a fluorophore (FAM) at the 5' end and a quenching agent (TAMRA) at the 3' end. Because of resonance energy transfer between the fluorophore and the quencher, no fluorescence is detected from free probe. When hybridized to its target sequence, the probe becomes susceptible to the 5' → 3' exonuclease activity of the polymerase used for amplification, releasing the fluorophore and freeing it from the influence of the quencher. Hence, as amplification proceeds, fluorescent output increases.

Immunoprecipitated DNA, obtained as described above, was used as template in a real-time amplification assay, using probe/primer sets specific for the integrated reporter gene containing four tandem VEGF 3a/1 binding sites (pGL-VFR) and the endogenous glyceraldehyde phosphate dehydrogenase (GAPDH) gene (which was used as a control for nonspecific effects of introduced ZFPs on cellular transcription and to control for nonspecific precipitation of chromatin by antibody or protein G beads). Sequences of the oligonucleotides used as primers and probes for detection of these genes are shown in Table 2. Standard curves were constructed for each gene-specific probe/primer set using a dilution series of genomic DNA template, and quantitation of VEGF and GAPDH sequences was accomplished using the relative quantitation method described by the manufacturer (PE Biosystems). Briefly, this method relates the Cₜ value obtained from the hydrolyzable probe analysis to template concentration, in arbitrary units. (The Cₜ value is the cycle number at which fluorescence first exceeds an arbitrary threshold value.) Cₜ values obtained for the various samples were converted to arbitrary units of template concentration, using the standard curve. Results are shown in Table 3. The first column identifies the plasmid that was introduced into the cells. The second and third columns provide values (in arbitrary units determined as described above) for the relative amount of immunoprecipitated DNA corresponding to the integrated reporter gene and the endogenous GAPDH gene, respectively. In the fourth column, the values for the integrated reporter gene are normalized to those obtained for the GAPDH gene, to control for sample-to-sample variability. In the final column, the GAPDH-normalized results for cells containing the non-ZFP plasmid (NVF) are assigned a value of 1.0, and the results obtained for cells containing a ZFP-expressing plasmid are expressed as enrichment of pGL sequences in the immunoprecipitate, compared to cells into which the NVF plasmid had been introduced.

The results indicate that integrated reporter sequences were enriched over 70-fold in immunoprecipitates from cells transfected with a construct encoding the six-finger VEGF 3a/1 protein, and over 10-fold in immunoprecipitates from cells in which the exogenous three-finger VEGF 1 protein was present. No enrichment was observed in cells containing a protein having a GATA 15.5 binding domain, which recognizes a target site different from those recognized by the VEGF 1 and VEGF 3a/1 proteins.

**Table 2: Primer and Probe sequences for hydrolyzable probe analysis**

| **Gene** | **Forward primer** | **Reverse primer** | **Probe** |
|---|---|---|---|
| VEGF | 5'-CTGGTAGCGG GGAGGATCG-3' (SEQ ID NO: 19) | 5'-GCCACGACCTCCG AGCTAC-3' (SEQ ID NO: 20) | 5'-CTACCCGGCT GCCCCAAGCCT C-3' (SEQ ID NO: 21) |
| pGL-VFR | 5'-CAAGTGCAGG TGCCAGAACA-3' (SEQ ID NO: 22) | 5'-CGGGACTATGGTTG CTGACT-3' (SEQ ID NO: 23) | 5'-CTACCCGGCT GCCCCAAGCCT C -3' (SEQ ID NO: 21) |
| GAPDH | 5'-CCTTTTGCAG ACCACAGTCCA-3 ' (SEQ ID NO: 24) | 5'-GCAGGGATGATGT TCTGGAGA-3' (SEQ ID NO: 25) | 5'-CACTGCCACC CAGAAGACTGT GG-3' (SEQ ID NO: 26) |

**Table 3: Analysis of chromatin immunoprecipitates by hydrolyzable probe assay**

| **Transfected construct** | **pGL (arbitrary units)** | **GAPDH (arbitrary units)** | **pGL/GAPDH** | **Enrichment *vs* NVF** |
|---|---|---|---|---|
| VEGF 3a/1 | 399 | 33.1 | 12.0 | 74.1 |
| VEGF 1 | 29.7 | 17.2 | 1.73 | 10.7 |
| GATA 15.5 | 6.70 | 47.1 | 0.142 | 0.88 |
| NVF | 2.06 | 12.7 | 0.162 | 1.0 |

### Example 12: Activation of an integrated reporter gene by an exogenous ZFP

To confirm the data obtained in Example 11, expression of the integrated reporter gene was assayed in the same samples in which the chromatin immunoprecipitation analysis was conducted. Since the exogenous ZFPs contained a VP16 activation domain, binding to their target site would be expected to result in increased expression of luciferase. Accordingly, luciferase activity was measured, as described in Example 10, for the samples described in Example 11, and the results are shown in Table 4. Luciferase expression was positively correlated with binding of exogenous ZFPs to pGL-VFR sequences. For example, the presence of the exogenous VEGF 3a/1 protein increased luciferase expression by 18-fold and VEGF 1 increased luciferase activity by almost 3-fold. These results are consistent with the data obtained by chromatin immunoprecipitation and provided additional evidence of ZFP binding to the integrated ZFP target sites.

**Table 4: Luciferase expression in cells transfected with ZFP-encoding plasmids**

| **Transfected construct** | **Luciferase activity*** |
|---|---|
| VEGF 3a/1 | 18 |
| VEGF 1 | 2.7 |
| GATA 15.5 | 0.8 |
| NVF | 1.0 |

| | |
|---|---|
| * Activity is expressed relative to the activity in cells transfected with a NVF-expressing plasmid. | |

### Example 13: Activation of endogenous VEGF gene by an exogenous ZFP

Activation of the integrated luciferase reporter gene containing VEGF target sites and immunoprecipitation of reporter sequences by the VEGF 1 and VEGF 3a/1 proteins, as shown in examples 10 and 11, provided evidence that these exogenous ZFPs are binding to their target sites in cellular chromatin. To investigate this question further, the expression of the endogenous VEGF gene was examined in cells containing exogenous VEGF 1 and VEGF 3a/1 proteins. Accordingly, the same samples that were analyzed in Examples 10 and 11 were assayed for endogenous VEGF mRNA (by real-time PCR analysis using reverse transcriptase-mediated PCR) and for VEGF protein (by ELISA). Results, normalized to the values obtained for cells transfected with the NVF plasmid, are shown in Table 5, and indicated that both the VEGF 1 and the VEGF 3a/1 ZFPs activated expression of VEGF mRNA and protein. The apparent activation of the endogenous VEGF gene by the GATA 15.5 ZFP is explained by the data obtained in Example 14, *infra.*

**Table 5: Expression of endogenous VEGF mRNA and protein in cells transfected with ZFP-encoding plasmids**

| **Transfected construct** | **VEGF mRNA (arbitrary units)** | **VEGF protein (arbitrary units)** |
|---|---|---|
| VEGF 3a/1 | 1.6 | 1.2 |
| VEGF 1 | 3.2 | 1.5 |
| GATA 15.5 | 2.0 | 1.2 |
| NVF | 1.0 | 1.0 |

### Example 14: Binding of exogenous ZFPs to an integrated reporter gene and the endogenous VEGF gene and effects on expression

Effects of exogenous ZFPs on integrated and endogenous genes containing VEGF target sites were analyzed by chromatin immunoprecipitation and reporter gene expression. In this example, immunoprecipitation of integrated and endogenous genes containing VEGF target sites were analyzed in the same experiment.

### Immunoprecipitation

Immunoprecipitated DNA, obtained as described in Example 11, was used as template in a real-time amplification assay. Three DNA targets were assayed: the integrated reporter gene containing four tandem VEGF 3a/1 binding sites (pGL-VFR), the endogenous VEGF gene, and the endogenous glyceraldehyde phosphate dehydrogenase (GAPDH) gene (which was used as a control for nonspecific precipitation).

Results of the analysis are shown in Table 6. The cells that were transfected contained both the endogenous VEGF gene and an integrated reporter gene (pGL-VFR) containing VEGF target sites. The first column of the table identifies the ZFP-encoding plasmid that was introduced into the cells. The second, third and fourth columns provide values (in arbitrary units determined as described above) for concentrations of immunoprecipitated DNA corresponding to the endogenous VEGF gene, the integrated reporter gene containing VEGF target sites and the endogenous GAPDH gene, respectively. In the fifth and sixth columns, the values obtained for the endogenous VEGF gene and the integrated VEGF-binding sequences were normalized to the values obtained for the endogenous GAPDH gene, to control for sample-to-sample variability.

The values obtained for the endogenous VEGF gene (VEGF) and for the integrated reporter containing VEGF target sites (pGL-VFR), normalized to the values obtained for GAPDH, were then normalized to the values obtained for cells transfected with NVF, a construct that lacks a zinc finger DNA-binding domain, to obtain a value for the degree to which VEGF sequences were enriched in immunoprecipitates from cells transfected with a construct encoding a ZFP. These values are shown in Table 7. The results indicate that sequences from the endogenous VEGF gene were enriched approximately 12-fold in immunoprecipitates from cells transfected with a construct encoding the six-finger VEGF 3a/1 protein, compared to cells transfected with a construct that lacks a zinc finger binding domain (NVF). Furthermore, sequences from an integrated reporter gene containing VEGF target sequences were enriched 170-fold by the VEGF 3a/1 protein and approximately 8-fold by the three-finger VEGF 1 protein.

**Table 6:**

| **Sequence analysis of chromatin immunoprecipitates by hydrolyzable probe assay** | | | | | |
|---|---|---|---|---|---|
| **Construct** | **VEGF (arbitrary units)** | **pGL-VFR (arbitrary units)** | **GAPDH (arbitrary units)** | **VEGF/ GAPDH** | **PGL-VFR/ GAPDH** |
| VEGF 3a/1 | 13,786.63 | 306.9 | 22.52 | 612.19 | 13.63 |
| VEGF 1 | 732.59 | 2.31 | 3.5 | 209.31 | 0.66 |
| GATA 15.5 | 9724.45 | 7.19 | 46.59 | 208.72 | 0.154 |
| NVF | 267.9 | 0.42 | 5.28 | 50.74 | 0.08 |
| Mock | 171.24 | 0.44 | 4.25 | 40.29 | 0.103 |

**Table 7: Sequence enrichment in immunoprecipitates**

| **Transfected Construct** | **Target Sequence** | |
|---|---|---|
| | **VEGF** | **pGL-VFR** |
| VEGF 3a/1 | 12.1 | 170.4 |
| VEGF 1 | 4.1 | 8.3 |
| GATA 15.5 | 4.1 | 1.9 |
| NVF | 1.0 | 1.0 |

In this experiment, both the six-finger and the three finger ZFPs promoted significant enrichment of both endogenous and integrated VEGF sequences in immunoprecipitates, compared to a protein lacking the zinc finger domain (NVF). Furthermore, the six-finger VEGF 3a/1 protein, when compared to a six-finger protein directed to a different target site (GATA 15.5) promoted enhanced immunoprecipitation of endogenous and integrated VEGF target sites. The date also indicate that the VEGF 1 and GATA 15.5 ZFPs bind equally well to the endogenous VEGF gene. This is consistent with the data obtained in Example 13, in which activation of endogenous VEGF mRNA and protein by GATA 15.5 was observed.

### Reporter gene expression

Analysis of luciferase expression (Table 8) revealed that the VEGF-binding ZFPs (VEGF 1 and VEGF 3a/1) stimulated reporter activity, compared to cells in which no exogenous ZFP was present (NVF). The GATA 15.5 ZFP did not stimulate reporter activity, consistent with the observation that GATA 15.5 showed very little immunoprecipitation of reporter sequences, compared to VEGF 1 and VEGF 3a/1 (Table 7). These results provide additional evidence of ZFP binding to the integrated ZFP target sites.

**Table 8: Luciferase expression in cells transfected with ZFP-encoding plasmids**

| **Transfected construct** | **Luciferase activity** |
|---|---|
| VEGF 3a/1 | 10 |
| VEGF 1 | 6 |
| GATA 15.5 | 1 |
| NVF | 1 |
| Mock | 1 |

### Expression of the endogenous VEGF gene

The production of mRNA and protein from the endogenous VEGF gene was assayed as described in Example 13, and the results are shown in Table 9. All ZFPs were observed to activate the endogenous VEGF gene, with VEGF 1 providing the highest levels of activation. The activation of the endogenous VEGF gene by GATA 15.5 is consistent with the ability of this protein to immunoprecipitate endogenous VEGF sequences (Table 7). This result points to a difference between the effects of GATA 15.5 on the endogenous VEGF gene and on the integrated VEGF reporter gene, which is neither strongly precipitated (Table 7) nor highly activated (Table 8) by GATA 15.5.

**Table 9: Expression of endogenous VEGF mRNA and protein in cells transfected with ZFP-encoding plasmids**

| **Transfected construct** | **VEGF mRNA (arbitrary units)** | **VEGF protein (arbitrary units)** |
|---|---|---|
| VEGF 3a/1 | 1.0 | 1.5 |
| VEGF 1 | 2.7 | 3.1 |
| GATA 15.5 | 2.0 | 2.0 |
| NVF | 1.0 | 1.0 |

A possible explanation for the apparent lack of VEGF transcriptional activation and low levels of VEGF protein production induced by VEGF 3a/1 is that tight binding of this six-finger ZFP counters, to a certain extent, its transcriptional activation potential.

### Example 15: Design of exogenous molecules that bind to the human Estrogen Receptor alpha (ER-α) gene

In this example, accessible regions in the chromatin of the human estrogen receptor-α (ER-α) gene were identified, an exogenous molecule comprising a zinc finger binding domain targeted to the accessible region was designed, the designed molecule was expressed in cells, and chromatin immunoprecipitation (ChIP) was used to demonstrate the binding of the designed molecule to its target sequence in the accessible region.

### Identification of accessible regions

An ER-positive breast carcinoma cell line, MCF-7, was used to identify DNase I hypersensitive regions (DHR) within an ~5kbp region of the Estrogen Receptor-α gene. Briefly, cells were grown to ~90% confluence in a T-225 flask, washed twice with PBS, harvested, and resuspended in a permeabilization buffer (10 mM Tris-HCl, pH 7.5, 10 mM NaCl, 60 mM KCl, 0.5 mM CaCl₂, 4.5 mM MgCl₂, 5% glycerol, 0.5 mM DTT, 0.5 mM PMSF, 0.5 % IGEPAL). After a 2.5 min incubation at room temperature, cells were centrifuged at 1000xg for 2.5 min, and separate aliquots of cells were resuspended in DNase I buffer (10 mM Tris-HCl, pH 7.5, 10 mM NaCl, 60 mM KCl, 0.5 mM CaCl₂, 4.5 mM MgCl₂, 5 % glycerol, 0.5 mM DTT, 0.5 mM PMSF, 0.5 % IGEPAL) containing increasing amounts of DNase I (0 to 12 Units/ml, DPRF grade, Worthington Biochemicals, Freehold, NJ) and incubated for 5 min at room temperature. The reactions were quenched by the addition of 0.5 M EDTA (to a final concentration of 10 mM) and buffer AL (Qiagen DNEASY kit, Valencia, CA). Genomic DNA was isolated using a Qiagen DNEASY kit and resolved on a 1% TAE-agarose gel, transferred to a nitrocellulose membrane and probed with estrogen receptor-α specific probes. Figure 4 shows the results, which indicate the presence of three DNase hypersensitive regions located at -320, -2100 and-3810, with respect to the proximal transcriptional startsite.

### Design of a ZFP targeted to an accessible region of the ER-α gene

An engineered fusion protein was designed to recognize a unique 9-base pair sequence in the DNase I hypersensitive region at-2 kb. This protein (BOS 3) comprised a nuclear localization sequence, a zinc finger binding domain, a KRAB repression domain and a FLAG epitope. The zinc finger binding domain was targeted to the sequence GGGGAGGAG, (SEQ ID NO: 27) which is complementary to the sequence CTCCTCCCC (SEQ ID NO: 28) in the coding strand. Zinc finger sequences (for amino acids -1 through +6 of the recognition helices) were RSDNLTR (SEQ ID NO: 29), RSDNLTR (SEQ ID NO: 30) and RSDALTK (SEQ ID NO: 31). Construction of a plasmid encoding the fusion protein and determination of the binding affinity of the zinc finger binding domain for its target sequence were performed according to methods disclosed in co-owned PCT WO 00/41566 and WO 00/42219. The dissociation constant (Kd) was determined to be 3.5 pM.

### Assay for binding of designed ZFPs

Cultures of MCF-7 cells were grown at 37°C in Dulbecco's modified Eagle's medium (Gibco BRL, Grand Island, NY/Rockville, MD) supplemented with glutamine, penicillin, streptomycin and 10% fetal bovine serum, to 50-65% confluence. They were then transfected with a plasmid encoding the BOS 3 fusion protein, using Lipofectamine 2000 (Gibco/BRL, Rockville, MD). Twenty-four hours after transfection, the medium was replaced with fresh medium. At 48 hours after transfection, when the cells had reached 80-90% confluence, formaldehyde was added to the culture medium to a final concentration of 1% (v/v). After 10 min at 37°C, the plate was washed with PBS to remove formaldehyde, cells were scraped from the plate, and suspended in PBS supplemented with a cocktail of protease inhibitors (0.5 mM PMSF, 20 ug/ml aprotinin, 20 ug/ml pepstatin, 20 ug/ml leupeptin). The cell suspension was then centrifuged at 1,000xg for 4 min at 4°C. Pelleted cells were resuspended in 0.2 ml of SDS lysis buffer supplemented with protease inhibitors (50 mM Tris-Cl, pH 8.1, 10 mM EDTA, 1% (w/v) sodium dodecyl sulfate, 0.5 mM PMSF, 20 ug/ml aprotinin, 20 ug/ml pepstatin, 20 ug/ml leupeptin). The resuspended cells were sonicated (10 five-second pulses on a VirSonic sonicator set at a power output of 4, with 10-second pauses between pulses), to lyse the cells and shear chromatin to an average DNA length of 200-500 nucleotide pairs. The sonicated lysate was centrifuged at 13,000 rpm for 10 min at 4°C, and the supernatant was recovered. 1.8 ml of ChIP buffer (16.7 mM Tris-Cl, pH 8.1, 1.2 mM EDTA, 167 mM NaCl, 1.1% Triton X-100, 0.01% SDS, 0.5 mM PMSF, 20 ug/ml aprotinin, 20 ug/ml pepstatin, 20 ug/ml leupeptin) was added to the cleared supernatant and 0.2 ml was removed as a pre-immunoprecipitation (pre-IP) input control. The input control sample was analyzed by agarose gel electrophoresis to verify that DNA fragments of 200-500 nucleotide pairs had been obtained.

The remainder of the sonicated lysate was pre-cleared by adding 0.1 ml of a 50% slurry of Protein A agarose beads (also containing salmon sperm DNA at 200 ug/ml), followed by gentle agitation for 90 min. The lysate was separated from the beads by centrifugation at 1,000xg for 5 min at 4°C. The cleared lysate was divided into two equal portions. To one portion, mouse monoclonal anti-FLAG antibody (IgG₁ isotype), obtained from Sigma Chemical Co. (St. Louis, MO), was added to a final concentration of 2 ug/ml of lysate, and the sample was incubated at 4°C overnight. 60 µl of a 50% slurry of protein A agarose beads (also containing 200 ug/ml salmon sperm DNA) was then added, and the sample was rotated for 60 min at 4°C.

Immune complexes were collected by centrifugation (2,000 rpm for 4 min at 4°C), and 250 µl of supernatant was retained as an unbound DNA control. The pelleted beads were washed as follows (each wash for 5 min at 4°C):
1. once with 20 mM Tris-Cl, pH 8.1, 1.2 mM EDTA, 150 mM NaCl, 1% Triton X-100, 0.1% SDS.
2. once with 20 mM Tris-Cl, pH 8.1, 1.2 mM EDTA, 500 mM NaCl, 1% Triton X-100, 0.1% SDS.
3. once with 10 mM Tris-Cl, pH 8.1, 1 mM EDTA, 250 mM LiCl, 1% sodium deoxycholate, 0.1 % NP-40
4. twice with 10 mM Tris-Cl, pH 8.0, 1 mM EDTA

Immune complexes were eluted from the beads by washing them twice with 0.25 ml of 1% SDS, 0.1 M NaHCO₃. For each wash, the elution buffer was added to the beads, they were mixed briefly by vortexing, then rotated at room temperature for 5 min. The eluates were combined, 20 µl of 5 M NaCl was added, and the sample was incubated at 65°C for 4 hrs to reverse formaldehyde crosslinks. A portion of the sample was then removed for protein analysis by Western blotting. To the remainder of the sample, 10 µl of 0.5 M EDTA, 20 µl of 1 M Tris-Cl, pH 6.5, and 5 µl of Proteinase K (20 mg/ml) were added, and the sample was incubated at 65°C for 30 min. DNA was recovered by phenol/chloroform extraction, followed by ethanol precipitation. The purified DNA was analyzed by real-time quantitative PCR, an assay known colloquially as "Taqman^{®}." The DNA was analyzed for the relative proportion of two sequences: (1) a region located 230 nucleotide pairs upstream of the BOS 3 binding site, and (2) a control sequence from the 18S rRNA gene. Primers and probes used in this assay are shown in Table 10.

**Table 10: Primers and probes for ChIP analysis of the ER-α gene**

| | **Sequence** | **SEQ. ID NO.** |
|---|---|---|
| **ER forward primer** | 5' -ACTGGCTGCTTCCCGAATC-3' | 32 |
| **ER reverse primer** | 5' -CGAGTGGCTCAGTGTGTGAACTA-3' | 33 |
| **ER probe** | 5'-CGCACAAACACATCCACACACTCTCTCTG-3' | 34 |
| **Control forward primer** | 5'-TTCCGATAACGAACGAGACTCT-3' | 35 |
| **Control reverse primer** | 5' -TGGCTGAACGCCACTTGTC-3' | 36 |
| **Control probe** | 5'-TAACTAGTTACGCGACCCCCGAG-3' | 37 |

The results, shown in Figure 5, show an approximately 20-fold enrichment of ER-alpha sequences associated with BOS3 in MCF-7 cells, compared to MCF-7 cells in which BOS3 was not expressed. Thus, chromatin immunoprecipitation indicates that an exogenous molecule, targeted to an accessible region of cellular chromatin, binds to its target site *in vivo*.

## Claims

1. A cell comprising a complex between an exogenous zinc finger DNA binding protein and chromosomal cellular chromatin;
wherein the exogenous zinc finger DNA binding protein is designed and/or selected to specifically bind to a target site in a region of the cellular chromatin that is sensitive to digestion with DNAse I; and
wherein the exogenous zinc finger DNA binding protein is bound to the target site.

2. The cell of claim 1, wherein the zinc finger DNA-binding protein is encoded by a nucleic acid introduced into the cell.

3. The cell of claim 1 or claim 2, wherein the cell is a plant cell.

4. The cell of claim 1 or claim 2, wherein the cell is an animal cell.

5. The cell of claim 4, wherein the cell is a human cell.

6. A method for forming a cell comprising a complex according to any of claims 1 to 5, wherein the method comprises:
introducing the zinc finger DNA-binding protein into the cell;
wherein the zinc finger DNA-binding protein binds to the target site region of cellular chromatin that is sensitive to digestion with DNAse I.

7. The method according to claim 6, wherein the binding site is in a coding region.

8. The method according to claim 6, wherein the binding site is in a noncoding region.

## Patentansprüche

1. Zelle, umfassend einen Komplex zwischen einem exogenen Zinkfinger-DNA-bindenden Protein und chromosomalem zellulären Chromatin;
wobei das exogene Zinkfinger-DNA-bindende Protein so konstruiert und/oder ausgewählt ist, dass es spezifisch an eine Zielstelle in einer Region des zelluläres Chromatins bindet, die empfindlich gegenüber einer Verdauung mit DNAse I ist; und
wobei das exogene Zinkfinger-DNA-bindende Protein an die Zielstelle gebunden ist.

2. Zelle nach Anspruch 1, wobei für das Zinkfinger-DNA-bindende Protein von einer in die Zelle eingeführten Nukleinsäure codiert wird.

3. Zelle nach Anspruch 1 oder Anspruch 2, wobei die Zelle eine pflanzliche Zelle ist.

4. Zelle nach Anspruch 1 oder Anspruch 2, wobei die Zelle eine tierische Zelle ist.

5. Zelle nach Anspruch 4, wobei die Zelle eine menschliche Zelle ist.

6. Verfahren zum Bilden einer Zelle, die einen Komplex nach einem der Ansprüche 1 bis 5 umfasst, wobei das Verfahren umfasst:
Einführen des Zinkfinger-DNA-bindenden Proteins in der Zelle;
wobei das Zinkfinger-DNA-bindende Protein an die Zielstellenregion des zelluläres Chromatins bindet, die empfindlich gegenüber einer Verdauung mit DNAse ist.

7. Verfahren nach Anspruch 6, wobei sich die Bindungsstelle in einer codierenden Region befindet.

8. Verfahren nach Anspruch 6, wobei sich die Bindungsstelle in einer nicht-kodierenden Region befindet.

## Revendications

1. Cellule contenant un complexe entre une protéine exogène de fixation à l'ADN en doigt de zinc et la chromatine cellulaire chromosomique ;
dans laquelle la protéine exogène de fixation à l'ADN en doigt de zinc est conçue et/ou sélectionnée pour se fixer de façon spécifique à un site cible dans une région de la chromatine cellulaire qui est sensible à la digestion avec la DNAse I ; et dans laquelle la protéine exogène de fixation à l'ADN en doigt de zinc est fixée au site cible.

2. Cellule de la revendication 1, dans laquelle la protéine de fixation à l'ADN en doigt de zinc est codée par un acide nucléique introduit dans la cellule.

3. Cellule de la revendication 1 ou de la revendication 2, dans laquelle la cellule est une cellule végétale.

4. Cellule de la revendication 1 ou de la revendication 2, dans laquelle la cellule est une cellule animale.

5. Cellule de la revendication 4, dans laquelle la cellule est une cellule humaine.

6. Procédé de formation d'une cellule contenant un complexe selon l'une quelconque des revendications 1 à 5, dans lequel le procédé comprend :
l'introduction de la protéine de fixation à l'ADN en doigt de zinc dans une cellule ;
la protéine de fixation à l'ADN en doigt de zinc se fixant à une région de site cible de la chromatine cellulaire qui est sensible à la digestion par la DNAse I.

7. Procédé selon la revendication 6, dans lequel le site de fixation est une région codante.

8. Procédé selon la revendication 6, dans lequel le site de fixation est une région non-codante.
